# EUROPÄISCHE PATENTANMELDUNG

(11) **EP 1 650 181 A1**
(43) Veröffentlichungstag der Anmeldung: **26.04.2006**
(21) Anmeldenummer: 05022109.2
(22) Anmeldetag: 11.10.2005
(51) Int. Cl.: C07C 43/11, C07C 43/10, C07C 41/18

(54) **Verfahren zur kontinuierlichen Herstellung von Alkylenglykoldiethern**

(30) Priorität: 21.10.2004 DE 102004051267
(71) Anmelder: Clariant Produkte (Deutschland) GmbH, 65929 Frankfurt am Main (DE)
(72) Erfinder: Stankowiak, Achim, Dr., 84503 Altötting (DE); Pfüller, Oliver, Dr., 65843 Sulzbach (DE); Schwarz, Sibylle, 84562 Mettenheim (DE); Snell, Alexander, Dr., 84503 Altötting (DE)

(57) **Zusammenfassung**

Gegenstand der Erfindung ist ein Verfahren zur Herstellung von Alkylenglykoldiethern der Formel (I) 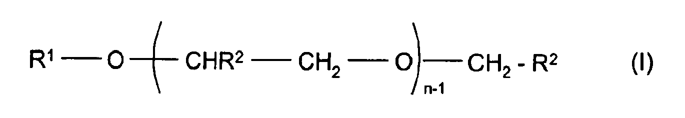 indem man Verbindungen der Formel (II) 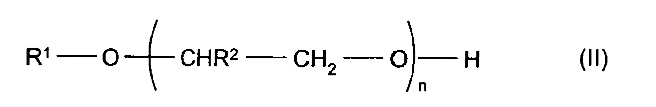 worin R¹ Wasserstoff oder C₁- bis C₃-Alkyl, R² Wasserstoff, CH₃ oder CH₂CH₃ und n eine Zahl von 1 bis 4 bedeuten, in flüssiger Phase in Gegenwart von Katalysatoren bei Temperaturen zwischen 170 und 300°C umsetzt, und die Verbindungen der Formel (I) kontinuierlich abdestilliert.

## Beschreibung

Die vorliegende Erfindung betrifft ein kontinuierliches Verfahren zur Herstellung von kettenförmigen Alkylenglykoldiethern.

Alkylenglykoldiether werden seit langer Zeit als polare, aprotische, inerte Lösungsmittel eingesetzt. Alkylenglykoldiether finden vor allem Anwendung als hochsiedende Lösungsmittel in der pharmazeutischen und chemischen Industrie, aber auch als Bindemittel in Lacken oder als Lösungsmittel in Reinigerformulierungen.

Zu ihrer Herstellung werden sogenannte indirekte Verfahren wie beispielsweise die Williamson-Ether-Synthese (K. Weissermel, H. J. Arpe "Industrielle Organische Chemie", 1998, Seite 179) oder die Hydrierung von Diglykoletherformal (DE-A-24 34 057) industriell angewandt oder beschrieben. Beide Verfahren haben allerdings Nachteile: die zweistufige Williamson-Ether-Synthese ist durch den stöchiometrischen Chlor- und Alkaliverbrauch, sowie die Abtrennung des Reaktionswassers und entstehenden Natriumchlorids wenig ökonomisch. Die Hydrierung von Formal wird unter Hochdruck durchgeführt, was hohe Investitionen in den Anlagenbau voraussetzt und daher nicht für geringere Produktionsmengen geeignet ist.

Bei den sogenannten direkten Verfahren wird Alkylenoxid in einen kettenförmigen Ether in Gegenwart von Lewis-Säuren wie BF₃ (US-4 146 736 und DE-A-26 40 505 in Verbindung mit DE-A-31 28 962) oder SnCl₄ (DE-A-30 25 434) insertiert. Der Nachteil dieser Verfahren besteht darin, dass unvermeidlich größere Mengen an cyclischen Nebenprodukten, wie beispielsweise Dioxan oder Dioxolan, gebildet werden.

Eine alternative Synthesemöglichkeit ist die katalytische Dehydrodecarbonylierung von Glykolen und Methylglykolen:

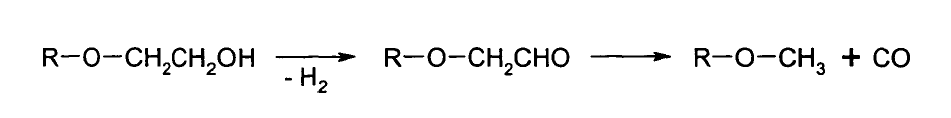

DE-A-29 00 279 beschreibt diesen Syntheseweg durch die Umsetzung von Polyethylenglykolen oder Polyethylenglykolmonomethylethern in der Gasphase bei 250-500°C in Gegenwart von geträgerten Palladium-, Platin-, Rhodium, Ruthenium- oder Iridium-Katalysatoren und Wasserstoff.

JP-A-600 28 429 beschreibt die Umsetzung von C₄- und längerkettigeren Monoalkylethern unter Verwendung eines Nickel/Rhenium-Katalysators, geträgert auf γ-Aluminiumoxid. Auch bei diesem Verfahren wird Wasserstoff zugeführt. Ebenfalls bekannt ist die Hydrierung von sekundären Hydroxylgruppen mit Wasserstoff bei Normaldruck unter Verwendung von Nickel-Trägerkatalysatoren (DE-A-38 02 783). Bei diesem Verfahren gelingt die Synthese ausdrücklich nicht, wenn Raney-Nickel verwendet wird.

Aus US-3 428 692 ist bekannt, dass durch Erhitzen von C₆- bis C₁₂-kettigen Monoalkyl- und Monophenylethern auf 200-300°C in Gegenwart von Nickel- und Cobalt-Katalysatoren die entsprechenden deformylierten methylblockierten Ethoxylate hergestellt werden können. Dabei entstehen jedoch Gemische von den gewünschten Methylethern mit nicht vollständig umgesetzten Ethoxylaten und 20 bis 30 % nicht identifizierten Aldehydverbindungen. EP-A-00 43 420 beschreibt ein ähnliches Verfahren unter Verwendung von Palladium-, Platin- oder RhodiumKatalysatoren, geträgert auf Al₂O₃ oder SiO₂.

Alle im derzeitigen Stand der Technik beschriebenen Verfahren sind entweder wenig selektiv oder aber technisch sehr aufwendig und daher unwirtschaftlich für die Herstellung von kurzkettigen Alkylenglykoldiethern. Die sich hieraus ergebende Aufgabe wurde erfindungsgemäß entsprechend den Angaben der Ansprüche gelöst.

Überraschenderweise können kurzkettige Alkylenglykole und Alkylenglykolmonoether in einem einfachen kontinuierlichen Verfahren durch Katalyse zu den gewünschten Alkylenglykoldiethern umgesetzt werden.

Gegenstand der Erfindung ist somit ein kontinuierliches Verfahren zur Herstellung von Alkylenglykoldiethern der Formel (I) 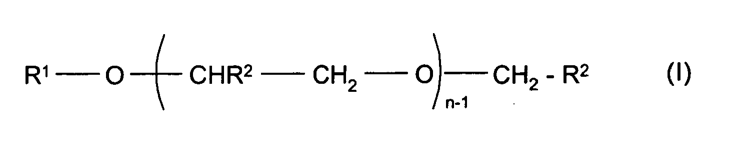 indem man Verbindungen der Formel (II) 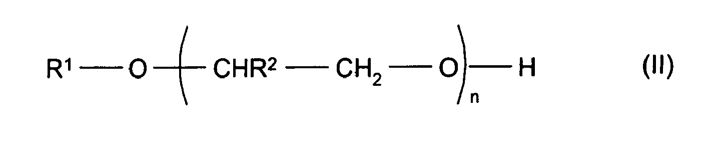 worin R¹ Wasserstoff oder C₁- bis C₃-Alkyl, R² Wasserstoff, CH₃ oder CH₂CH₃ und n eine Zahl von 1 bis 4 bedeuten, in flüssiger Phase in Gegenwart von Katalysatoren bei Temperaturen zwischen 170 und 300°C umsetzt, und die Verbindungen der Formel (I) kontinuierlich abdestilliert.

Als Ausgangsstoffe eignen sich sowohl reine Ethoxylate, Propoxylate oder Butoxylate als auch die aus Ethylenoxid, Propylenoxid und/oder Butylenoxid aufgebauten gemischten Alkoxylate sowie Monoalkylalkoxylate basierend auf Ethylenoxid, Propylenoxid und/oder Butylenoxid. R¹ bedeutet vorzugsweise H oder Methyl.
R² bedeutet vorzugsweise Wasserstoff und n ist vorzugsweise eine Zahl von 1 bis 3. Besonders bevorzugt setzt man Methyldiglykol und Methyltriglykol ein. Der Ausgangsstoff gemäß Formel II wird der Reaktion kontinuierlich zugeführt, während das entstandene Produkt gleichzeitig kontinuierlich abgeführt wird, beispielsweise durch Destillation.

Als Katalysatoren eignen sich vorzugsweise reine Nickelkatalysatoren sowie Mischungen von Nickel (auch Raney-Nickel) mit anderen Metallen wie beispielsweise Palladium, Platin, Cobalt, Rhodium, Iridium, Eisen, Ruthenium, Osmium, Mangan, Rhenium, Chrom, Molybdän, Kupfer (auch Raney-Kupfer) oder Bismut. Diese Mischungen können sowohl heterogener Natur sein, als auch Dotierungen auf der Katalysatoroberfläche. Das Trägermaterial ist nicht kritisch. Geeignete Trägermaterialien sind beispielsweise Aluminium- und andere Metalloxide, Kohle, Kieselgur, Siliciumdioxid, Siliciumcarbid, Zeolithe und ähnliche. Bevorzugt setzt man eine Mischung von Raney-Nickel mit Palladium auf Aktiv-Kohle ein. Die einzusetzende Katalysatormenge kann in weiten Grenzen variieren. In der Regel wird soviel Katalysator eingesetzt, dass 0,2 bis 15 Gew.-% Katalysator bezogen auf die Einsatzmenge der Verbindung der Formel (I), vorzugsweise 0,5 bis 10 Gew.-% Katalysator, bezogen auf die Einsatzmenge der Verbindung der Formel (I), vorliegen.

Die Umsetzung an den Katalysatoren erfolgt vorzugsweise bei 180 bis 250°C. Die Reaktion führt man im allgemeinen bei Normaldruck durch, man kann jedoch auch im Unter- oder Überdruck arbeiten. Bei dem erfindungsgemäßen Verfahren kann Wasserstoff oder ein inertes Gas als Trägergas verwendet werden.

Das erfindungsgemäße Verfahren wird nun an einigen Beispielen noch näher erläutert:

### Beispiel 1: Darstellung von Monoethylenglykoldimethylether

In einem 10-Liter-Rührautoklav, ausgestattet mit beheizbarem Brüdenrohr, wurden 5,0 kg Methyldiglykol (41,7 mol), 624 g Pd-Katalysator (5 % auf Aktivkohle) und 150 g Raney-Nickel vorgelegt. Der Ansatz wurde bei einem Überdruck von 3,0 bar langsam auf 220°C aufgeheizt und die sich bildenden Produkte kontinuierlich über das Brüdenrohr abdestilliert. Gleichzeitig wurde Methyldiglykol kontinuierlich zudosiert, so dass der Füllstand im Reaktor annähernd konstant blieb. Nach der Destillation über das Brüdenrohr erhielt man ein Produktgemisch bestehend aus 75 % Monoethylenglykol-dimethylether, 11 % Diethylenglykoldimethylether, 2 % Triethylenglykoldimethylether, 10 % Methylglykol und 2 % weiterer Nebenprodukte. Durch eine zweite Destillation über eine Kolonne (18 theoretische Böden, Rücklaufverhältnis 1:3) werden diese Produkte voneinander getrennt und Methyldiglykol rückgeführt. Der Versuch wird nach 10 Stunden beendet.
Die Ausbeute an Monoethylenglykoldimethylether betrug 995,8 g/h.

### Beispiel 2: Darstellung von Diethylenglykoldimethylether

Verfahren analog Beispiel 1 mit den folgenden Änderungen:
5,0 kg Methyltriglykol (30,5 mol), 624 g Pd-Katalysator (5 % auf Aktivkohle) und 150 g Raney-Nickel.
   Das Produktgemisch bestand aus 74 % Diethylenglykoldimethylether, 20 % Methyltriglykol, 4 % Methyldiglykol und 2 % weitere Nebenprodukte.
   Die Ausbeute an Diethylenglykoldimethylether betrug 1839,6 g/h.

### Beispiel 3: Darstellung von Diethylenglykoldimethylether

In einem 250 mL Reaktionskolben, ausgestattet mit Rührer, Stromstörer, Tropftrichter und Destillationsbrücke wurden 180,2 g (1,1 mol) Methyltriglykol, 9,7 g Palladiumkatalysator (5 %m/m Palladium auf Aktivkohle) und 3,7 g Raney-Nickelkatalysator vorgelegt. Die vorgelegte Mischung wurde unter Rühren auf 220°C temperiert. Die sich bildenden Produkte wurden kontinuierlich über die Destillationsbrücke abdestilliert. Parallel wurde das Edukt Methyltriglykol kontinuierlich über einen Tropftrichter so zudosiert, dass das Verhältnis zugetropftes Methyltriglykol zu abdestilliertem Produkt konstant blieb.
Das Produktgemisch bestand aus 67 % Diethylenglykoldimethylether, 25 % Methyltriglykol, 5 % Methyldiglykol und 3 % weiterer Nebenprodukte.
Die Ausbeute an Diethylenglykoldimethylether betrug 70,8 g/h.

### Beispiel 4: Darstellung von Diethylenglykoldimethylether

Verfahren analog Beispiel 3 mit den folgenden Änderungen:
180,5 g (1,1 mol) Methyltriglykol und 2,8 g Raney-Nickelkatalysator.
   Das Produktgemisch bestand aus 52 % Diethylenglykoldimethylether, 20 % Methyltriglykol, 21 % Methyldiglykol und 7 % weiterer Nebenprodukte.
   Die Ausbeute an Diethylenglykoldimethylether betrug 33,1 g/h.

### Beispiel 5: Darstellung von Diethylenglykoldimethylether

Verfahren analog Beispiel 3 mit den folgenden Änderungen:
182,6 g (1,12 mol) Methyltriglykol und 3,4 g Raney-Nickelkatalysator und 3,4 g Raney-Kupferkatalysator.
   Das Produktgemisch bestand aus 56 % Diethylenglykoldimethylether, 13 % Methyltriglykol, 20 % Methyldiglykol und 11 % weiterer Nebenprodukte.
   Die Ausbeute an Diethylenglykoldimethylether betrug 25,2 g/h.

### Beispiel 6: Darstellung von Diethylenglykoldimethylether

Verfahren analog Beispiel 3 mit den folgenden Änderungen:
180,6 g (1,1 mol) Methyltriglykol und 1,5 g Raney-Nickel, 2,7 g Palladium und 0,9 g Rhodium.
   Das Produktgemisch bestand aus 68 % Diethylenglykoldimethylether, 17 % Methyltriglykol, 10 % Methyldiglykol und 5 % weiterer Nebenprodukte.
   Die Ausbeute an Diethylenglykoldimethylether betrug 25,1 g/h.

### Beispiel 7: Darstellung von Diethylenglykoldimethylether

Verfahren analog Beispiel 3 mit den folgenden Änderungen:
180,1 g (1,1 mol) Methyltriglykol und 1,4 g Raney-Nickel, 2,7 g Palladium und 0,9 g Rhenium.
   Das Produktgemisch bestand aus 63 % Diethylenglykoldimethylether, 10 % Methyltriglykol, 16 % Methyldiglykol und 11 % weiterer Nebenprodukte.
   Die Ausbeute an Diethylenglykoldimethylether betrug 20,1 g/h.

### Beispiel 8: Darstellung von Diethylenglykoldimethylether

Verfahren analog Beispiel 3 mit den folgenden Änderungen:
181,1 g (1,1 mol) Methyltriglykol und 1,9 g Raney-Nickel und 2,5 g Platin dotiert mit Bismut.
   Das Produktgemisch bestand aus 54 % Diethylenglykoldimethylether, 16 % Methyltriglykol, 21 % Methyldiglykol und 9 % weiterer Nebenprodukte.
   Die Ausbeute an Diethylenglykoldimethylether betrug 26,6 g/h.

## Patentansprüche

1. Kontinuierliches Verfahren zur Herstellung von Alkylenglykoldiethern der Formel (I) 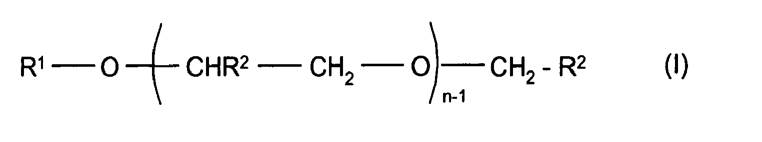 indem man Verbindungen der Formel (II) 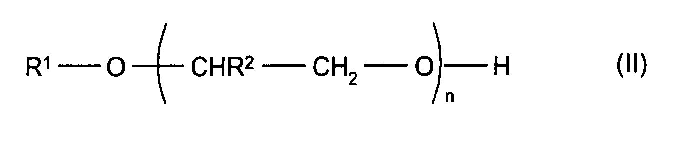 worin R¹ Wasserstoff oder C₁- bis C₃-Alkyl, R² Wasserstoff, CH₃ oder CH₂CH₃ und n eine Zahl von 1 bis 4 bedeuten, in flüssiger Phase in Gegenwart von Katalysatoren bei Temperaturen zwischen 170 und 300°C umsetzt, und die Verbindungen der Formel (I) kontinuierlich abdestilliert.

2. Verfahren nach Anspruch 1, worin R¹ für H oder Methyl steht.

3. Verfahren nach Anspruch 1 und/oder 2, worin R² für H steht.

4. Verfahren nach einem oder mehreren der Ansprüche 1 bis 3, worin n für eine Zahl von 1 bis 3 steht.

5. Verfahren nach einem oder mehreren der Ansprüche 1 bis 4, worin der Katalysator Nickel enthält.

6. Verfahren nach einem oder mehreren der Ansprüche 1 bis 5, worin der Katalysator ein reiner Nickelkatalysator ist.

7. Verfahren nach einem oder mehreren der Ansprüche 1 bis 6, worin der Katalysator neben Nickel ein oder mehrere Metalle ausgewählt aus Pd, Pt, Co, Rh, Ir, Fe, Rn, Os, Mn, Re, Cr, Mo, Cu oder Bi umfasst.

8. Verfahren nach einem oder mehreren der Ansprüche 1 bis 7, worin die Menge an Katalysator 0,2 bis 15 Gew.-% bezogen auf die Menge der Verbindung der Formel (I) beträgt.
